# EUROPEAN PATENT APPLICATION

(11) **EP 4 675 162 A1**
(43) Date of publication of application: **07.01.2026**
(21) Application number: 24778808.6
(22) Date of filing: 16.02.2024
(51) Int. Cl.: F21V 9/38, F21S 2/00, F21V 8/00, F21V 9/35, F21Y 115/10, F21Y 115/30

(54) **LIGHT SOURCE DEVICE**

(30) Priority: 28.03.2023 JP 2023051786
(71) Applicant: Sony Group Corporation, Tokyo 108-0075 (JP)
(72) Inventor: HOMMA, Keisuke, Tokyo 108-0075 (JP); ASANO, Yoshiro, Tokyo 108-0075 (JP); MIYAO, Ryo, Tokyo 108-0075 (JP)
(74) Representative: D Young & Co LLP
(86) International application number: PCT/JP2024/005413
(87) International publication number: WO 2024/202663

(57) **Abstract**

A light source apparatus according to one embodiment of the present disclosure includes: a light source unit that divides excitation light into a plurality of light beams and outputs the plurality of light beams; a light guide member including, on an inner side thereof, a hollow light guide path having a first opening and a second opening on a side opposite to the first opening, the light guide member having a plurality of wavelength converters provided on at least a portion of a side surface of the light guide path, the plurality of wavelength converters being configured to emit light beams having wavelength bands different from each other; and a light condenser device that guides each of the plurality of light beams output from the light source unit to a corresponding one of the plurality of wavelength converters.

## Description

### LIGHT SOURCE APPARATUS

### Technical Field

The present disclosure relates to, for example, a light source apparatus including an IR light source as a light source.

### Background Art

For example, Patent Literature 1 discloses a light source apparatus in which, on an optical path of light output from a plurality of solid-state light sources, a light condenser lens, a dichroic mirror, and a light pipe are disposed in the stated order. The light pipe includes a pair of openings (a first opening and a second opening), and a phosphor is disposed adjacent to the second opening on a side opposite to the dichroic mirror side. Light fluxes (excitation light beams) output from the plurality of solid-state light sources are condensed to predetermined locations by the light condenser lens to be reflected by the dichroic mirror, and enter the light pipe from the first opening. The light fluxes that have entered the light pipe pass through the inside of the light pipe to irradiate the phosphor disposed adjacent to the second opening. The phosphor generates light (illumination light) having a predetermined wavelength band. The illumination light generated from the phosphor advances in a direction opposite to the excitation light, and is output from the first opening to pass through the dichroic mirror to be extracted in a direction different from the light fluxes output from the plurality of solid-state light sources.

### Citation List

### Patent Literature

Patent Literature 1: Japanese Unexamined Patent Application Publication No. 2014-010181

### Summary of the Invention

Incidentally, for example, in a light source apparatus used in medical equipment, a high color rendering property is demanded.

It is thus desirable to provide a light source apparatus having a high color rendering property.

A light source apparatus according to one embodiment of the present disclosure includes: a light source unit that divides excitation light into a plurality of light beams and outputs the plurality of light beams; a light guide member including, on an inner side thereof, a hollow light guide path having a first opening and a second opening on a side opposite to the first opening, the light guide member having a plurality of wavelength converters provided on at least a portion of a side surface of the light guide path, the plurality of wavelength converters being configured to emit light beams having wavelength bands different from each other; and a light condenser device that guides each of the plurality of light beams output from the light source unit to a corresponding one of the plurality of wavelength converters.

In the light source apparatus according to one embodiment of the present disclosure, by using the light source unit that outputs the plurality of divided light beams and the light guide member including, on the inner side thereof, the hollow light guide path having the first opening and the second opening on a side opposite to the first opening and having the plurality of wavelength converters that emits light beams having wavelength bands different from each other provided in at least a portion of the side surface of the light guide path, each of the plurality of divided light beams output from the light source unit as the excitation light is applied via the light condenser device to a corresponding one of the plurality of wavelength converters provided on the inner side of the light guide member. This allows light having a desired wavelength distribution to be extracted.

### Brief Description of the Drawings

[FIG. 1] FIG. 1 is a schematic diagram illustrating a configuration example of a light source apparatus according to an embodiment of the present disclosure.
[FIG. 2] FIG. 2 is a schematic diagram illustrating an example of a structure of a light source unit illustrated in FIG. 1.
[FIG. 3] FIG. 3 is an explanatory exploded perspective diagram illustrating an example of a configuration of the light source unit illustrated in FIG. 1.
[FIG. 4] FIG. 4 is an explanatory perspective diagram illustrating an example of a configuration of a light pipe illustrated in FIG. 1.
[FIG. 5] FIG. 5 is an explanatory diagram illustrating a relationship between a size of an opening of the light pipe and a spot size of excitation light entering the light pipe.
[FIG. 6] FIG. 6 is an explanatory diagram illustrating a position of a phosphor layer disposed within a light guide path of the light pipe.
[FIG. 7A] FIG. 7A is an angle distribution diagram of the excitation light entering the light pipe illustrated in FIG. 1.
[FIG. 7B] FIG. 7B is an illuminance distribution diagram of illumination light entering a light guide illustrated in FIG. 1.
[FIG. 8] FIG. 8 is an angle distribution diagram of the illumination light output from an exit opening of the light pipe illustrated in FIG. 1.
[FIG. 9] FIG. 9 is an angle distribution diagram of the illumination light output from the exit opening of the light pipe illustrated in FIG. 1 via a diffusion plate.
[FIG. 10] FIG. 10 is a wavelength distribution diagram of illumination light extracted from a general light source apparatus.
[FIG. 11] FIG. 11 is a wavelength distribution diagram of illumination light extracted from the light source apparatus illustrated in FIG. 1.
[FIG. 12] FIG. 12 is a schematic diagram illustrating a configuration example of a light source apparatus according to Modification Example 1 of the present disclosure.
[FIG. 13] FIG. 13 is a schematic diagram illustrating a configuration example of a light source apparatus according to Modification Example 2 of the present disclosure.
[FIG. 14] FIG. 14 is a schematic diagram illustrating a configuration example of a light source apparatus according to Modification Example 3 of the present disclosure.
[FIG. 15A] FIG. 15A is an explanatory diagram illustrating a configuration example of a phosphor layer disposed in a light guide path of a light pipe according to Modification Example 4 of the present disclosure.
[FIG. 15B] FIG. 15B is an explanatory diagram illustrating a configuration example of a phosphor layer disposed in the light guide path of the light pipe according to Modification Example 4 of the present disclosure.
[FIG. 16] FIG. 16 is an explanatory diagram illustrating a configuration example of phosphor layers disposed in the light guide path of the light pipe according to Modification Example 4 of the present disclosure.

### Modes for Carrying Out the Invention

Hereinafter, an embodiment for practicing the present disclosure is described in detail with reference to the drawings. The following description is a specific example of the present disclosure, and the present disclosure is not limited to the following embodiment. Moreover, the present disclosure does not limit the disposition, dimensions, dimension ratios, and the like of respective components illustrated in the drawings thereto. It is to be noted that description is given in the following order.
1. Embodiment (an example of a light source apparatus including a light source unit that is possible to divide light beams and a light pipe including a wavelength converter on a side surface of a light guide path)
2. Modification Examples
   2-1. Modification Example 1 (another example of the configuration of the light source apparatus)
   2-2. Modification Example 2 (another example of the configuration of the light source apparatus)
   2-3. Modification Example 3 (another example of the configuration of the light source apparatus)
   2-4. Modification Example 4 (another example of a configuration of the wavelength converter)

### <1. Embodiment>

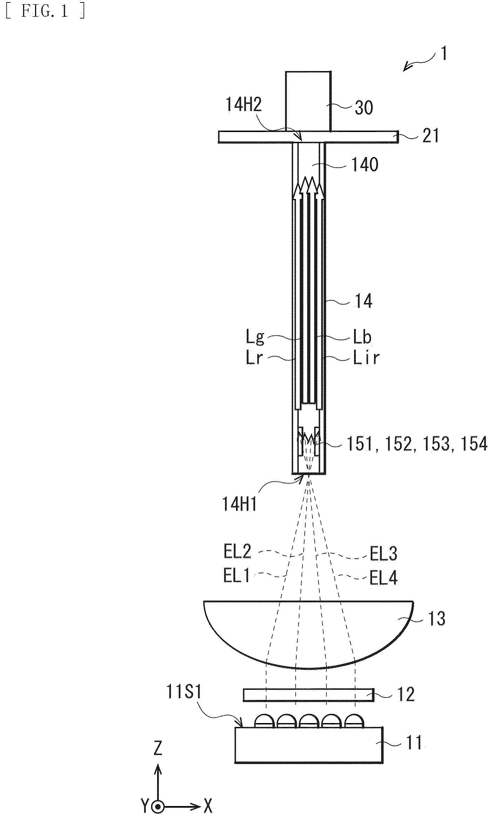

FIG. 1 is a diagram illustrating a configuration example of a light source apparatus (a light source apparatus 1) according to an embodiment of the present disclosure. The light source apparatus 1 is used as, for example, a light source of medical equipment such as a gastroscopy camera or an endoscope camera. The light source apparatus 1 of the present embodiment includes: a light source unit 11 that divides excitation light into a plurality of light beams and outputs the plurality of light beams; a light pipe 14 including, on an inner side thereof, a hollow light guide path 140, the light pipe 14 including a plurality of phosphor layers (a red phosphor layer 151, a green phosphor layer 152, a blue phosphor layer 153, and a near infrared (Ir) phosphor layer 154) provided on a portion of a side surface of the light guide path 140, the plurality of phosphor layers being configured to emit light beams having wavelength bands different from each other; and a light condenser lens 13 disposed between the light source unit 11 and the light pipe 14. Each of the plurality of light beams (excitation light beams EL1, EL2, EL3, and EL4) output from the light source unit 11 irradiates, via the light condenser lens 13, a corresponding one of the red phosphor layer 151, the green phosphor layer 152, the blue phosphor layer 153, and the IR phosphor layer 154 provided on the side surface of the light guide path 140. This makes it possible to extract illumination light having a desired wavelength distribution from the light pipe 14.

### [Configuration of Light Source Apparatus]

The light source apparatus 1 includes, for example, the light source unit 11, the light condenser lens 13, the light pipe 14, and a wavelength converter 15. The light source apparatus 1 further includes a beam shaping device 12, a diffusion plate 21, and a light guide 30.

Here, the light source unit 11 corresponds to a specific example of a "light source unit" in the embodiment of the present disclosure. The light pipe 14 corresponds to a specific example of a "light guide member" in the embodiment of the present disclosure. The red phosphor layer 151, the green phosphor layer 152, the blue phosphor layer 153, and the IR phosphor layer 154 correspond to a specific example of a "plurality of wavelength converters" in the embodiment of the present disclosure. The light condenser lens 13 corresponds to a specific example of a "light condenser device" in the embodiment of the present disclosure.

The members configuring the above-described light source apparatus 1 are disposed as follows. The light source unit 11 is disposed to output the plurality of output light beams in a Z-axis direction. In the output direction of the light source unit 11 (the Z-axis direction), the beam shaping device 12, the light condenser lens 13, the light pipe 14, the diffusion plate 21, and the light guide 30 are disposed in the stated order. The plurality of phosphor layers (the red phosphor layer 151, the green phosphor layer 152, the blue phosphor layer 153, and the near infrared (Ir) phosphor layer 154) is provided on the inner side surface of the light pipe 14.

The light source unit 11 is a light source that is possible to output a plurality of divided light beams. Specifically, the light source unit 11 is configured to output divided light beams corresponding to the number of phosphor layers provided on the side surface of the light guide path 140 on the inner side of the light pipe 14 disposed in a stage following the light source unit 11.

Although details are described later, for example, the light pipe 14 includes, on the inner side thereof, the hollow light guide path 140 having four side surfaces 140S1, 140S2, 140S3, and 140S4 that are opposed to each other vertically and horizontally, and each of the red phosphor layer 151, the green phosphor layer 152, the blue phosphor layer 153, and the IR phosphor layer 154 is provided on a corresponding one of the four side surfaces 140S1, 140S2, 140S3, and 140S4. The phosphor layers 151, 152, 153, and 154 are respectively irradiated with the independent excitation light beams EL1, EL2, EL3, and EL4. That is, the light source unit 11 outputs four independent light beams (the excitation light beams EL1, EL2, EL3, and EL4) that respectively irradiate the four types of phosphor layers 151, 152, 153, and 154.

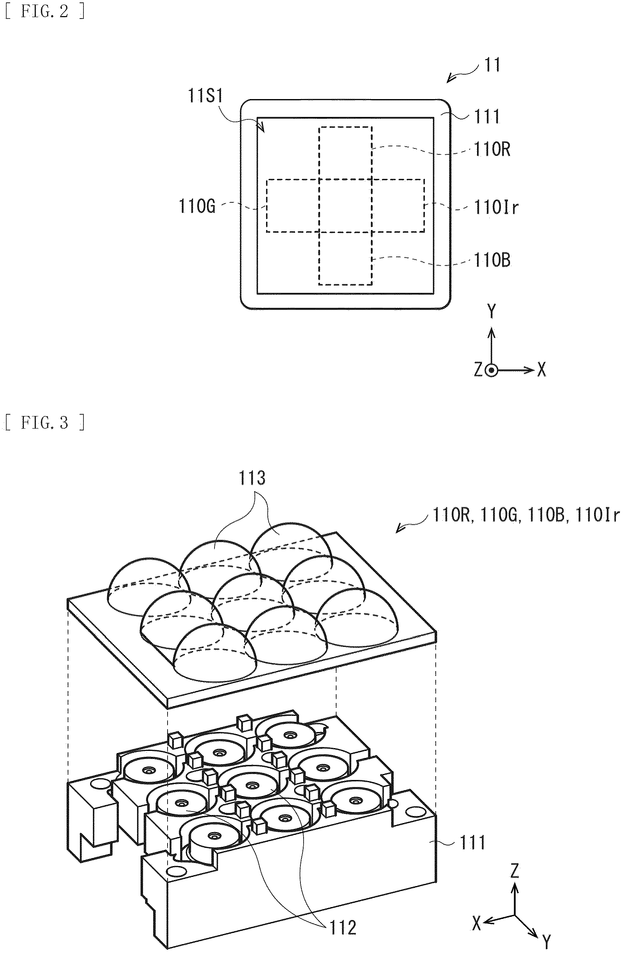

For example, the light source unit 11 includes, as illustrated in FIG. 2, four light source blocks (a red light source block 110R, a green light source block 110G, a blue light source block 110B, and an IR light source block 110Ir). The red light source block 110R outputs a light beam (the excitation light EL1) that irradiates the red phosphor layer 151. The green light source block 110G outputs a light beam (the excitation light EL2) that irradiates the green phosphor layer 152. The blue light source block 110B outputs a light beam (the excitation light EL3) that irradiates the blue phosphor layer 153. The IR light source block 110Ir outputs a light beam (the excitation light EL4) that irradiates the IR phosphor layer 154.

The light source blocks 110R, 110G, 110B, and 110Ir are disposed at positions respectively corresponding to the red phosphor layer 151, the green phosphor layer 152, the blue phosphor layer 153, and the IR phosphor layer 154 to be described later. For example, as illustrated in FIG. 1, in a case where one light condenser lens 13 is disposed between the light source unit 11 and the light pipe 14, at the time of opposing an output surface 11S1 of the light source unit 11 and a light entrance (an opening 14H1) for the excitation light beams EL1, EL2, EL3, and EL4 of the light pipe 14 to each other, the light source blocks 110R, 110G, 110B, and 110Ir are disposed in such a manner that formation positions thereof are reversed vertically and horizontally from the red phosphor layer 151, the green phosphor layer 152, the blue phosphor layer 153, and the IR phosphor layer 154 provided on the respective four side surfaces 140S1, 140S2, 140S3, and 140S4 configuring the light guide path 140 of the light pipe 14. That is, in the light pipe 14, as viewed from the opening 14H1 side, the blue phosphor layer 153 is provided on an upper side surface 140S3 of the light guide path 140, the red phosphor layer 151 is provided on a lower side surface 140S1 thereof, the green phosphor layer 152 is provided on a left side surface 140S2 thereof, and the IR phosphor layer 154 is provided on a right side surface 140S4 thereof. In contrast, in the light source unit 11, as viewed from the output surface 11S1 side, the red light source block 110R is disposed on the upper side, the IR light source block 110Ir is disposed on the lower side, the green light source block 110G is disposed on the left side, and the blue light source block 110B is disposed on the right side. This allows the excitation light EL1 output from the red light source block 110R to selectively irradiate the red phosphor layer 151 via the light condenser lens 13. The excitation light EL2 output from the green light source block 110G selectively irradiates the green phosphor layer 152 via the light condenser lens 13. The excitation light EL3 output from the blue light source block 110B selectively irradiates the blue phosphor layer 153 via the light condenser lens 13. The excitation light EL4 output from the IR light source block 110Ir selectively irradiates the IR phosphor layer 154 via the light condenser lens 13.

The red light source block 110R, the green light source block 110G, the blue light source block 110B, and the IR light source block 110Ir are independently controlled by currents. That is, it is possible to independently switch ON/OFF of each of the light source blocks 110R, 110G, 110B, and 110Ir, and also independently control the intensities of the excitation light beams EL1, EL2, EL3, and EL4 irradiating the respective phosphor layers 151, 152, 153, and 154. This makes it possible to freely adjust a wavelength distribution of light (illumination light) extracted from the light pipe 14. Further, it is possible to adjust light deteriorated due to elapse of time.

Each of the light source blocks 110R, 110G, 110B, and 110Ir includes, as a light source, one or a plurality of solid-state light emitting devices 112 that outputs light of a predetermined wavelength band. The one or the plurality of solid-state light emitting devices 112 is disposed in, for example, an array shape on a base portion 111. FIG. 3 is an exploded perspective diagram illustrating an example of the configuration of the light source unit 11. In the light source unit 11 illustrated in FIG. 1, for example, ten solid-state light emitting devices 112 are disposed on the base portion 111 in, for example, three rows and three columns.

The base portion 111 supports the plurality of solid-state light emitting devices 112, and promotes heat dissipation of the plurality of solid-state light emitting devices 112 that has generated heat by light emission. It is thus preferred that the base portion 111 be formed by using a material having a high heat conductivity, and is formed by using, for example, aluminum (Al), copper (Cu), iron (Fe), or the like.

Examples of the plurality of solid-state light emitting devices 112 include semiconductor lasers (Laser Diodes: LDs). For example, LDs that oscillate laser light (excitation light EL: blue light Lb) of a wavelength band corresponding to blue having a wavelength of 400 nm to 470 nm are used as the plurality of solid-state light emitting devices 112. The laser light output from the plurality of solid-state light emitting devices 112 is polarized to S polarization or P polarization, and, here, is polarized to P polarization, for example. As another example, light emitting diodes (LEDs) may be used as the plurality of solid-state light emitting devices 112.

A plurality of lenses 113 is disposed above the plurality of solid-state light emitting devices 112. Each of the plurality of lenses 113 is, for example, a collimator lens. The plurality of lenses 113 adjusts the laser light (the excitation light EL) output from each of the plurality of solid-state light emitting devices 112 into parallel light and outputs the parallel light. For example, the plurality of lenses 113 is disposed in an array shape as illustrated in FIG. 3, and is, for example, fixed to the base portion 111 via an adhesive.

The beam shaping device 12 outputs laser light with the angle distribution of the incident laser light being adjusted. For example, laser light has a large radiation angle difference between the slow axis (H direction) and the fast axis (V direction), and has an angle distribution that is greatly biased at an LD light emitting point. The beam shaping device 12 outputs, to the condenser lens 13, the laser light (the excitation light beams EL1, EL2, EL3, and EL4) output from the light source unit 11 with the bias of the angle distribution thereof being reduced.

The light condenser lens 13 is disposed between the beam shaping device 12 and the light pipe 14, and condenses the excitation light beams EL1, EL2, EL3, and EL4 that have been reduced in bias of the angle distribution by the beam shaping device 12 toward the light pipe 14. The light condenser lens 13 is preferably formed by using, for example, a high refractive index material. As another example, the light condenser lens 13 may have a configuration of a combination of a plurality of lenses. This makes it possible to decrease the size of the light condensing point and allow the excitation light beams EL1, EL2, EL3, and EL4 entering the light pipe 14 to efficiently irradiate the respective corresponding phosphor layers 151, 152, 153, and 154.

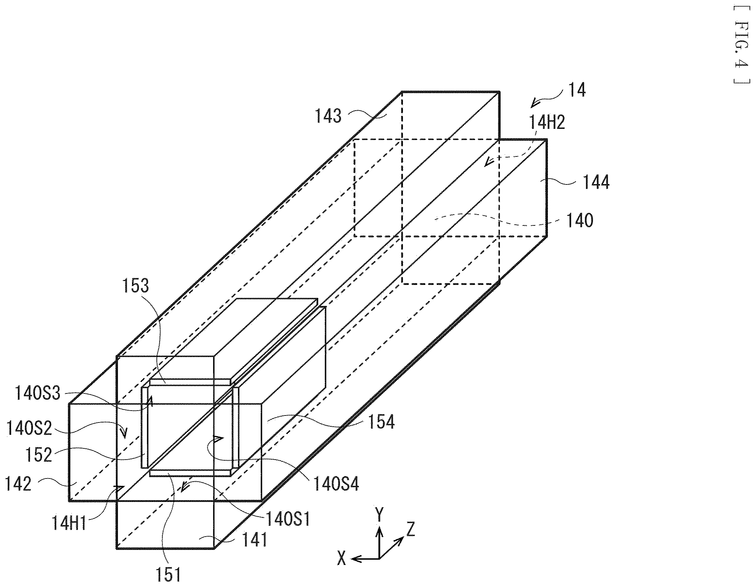

The light pipe 14 is a light guide member having a pair of opposing openings 14H1 and 14H2, and including, on an inner side thereof, the hollow light guide path 140. Specifically, for example, as illustrated in FIG. 4, the light pipe 14 is a columnar light guide member that is obtained by combining four glass substrates 141, 142, 143, and 144, and includes, between the opening 14H1 and the opening 14H2 each having a rectangular shape, the light guide path 140 surrounded by the four side surfaces 140S1, 140S2, 140S3, and 140S4 that are opposed to each other vertically and horizontally. On each of the four side surfaces 140S1, 140S2, 140S3, and 140S4, for example, a metal film such as an aluminum film is formed to serve as a reflection surface.

The red phosphor layer 151, the green phosphor layer 152, the blue phosphor layer 153, and the IR phosphor layer 154 are respectively provided on the four side surfaces 140S1, 140S2, 140S3, and 140S4 configuring the light guide path 140. The red phosphor layer 151 absorbs the excitation light EL1 output from the light source unit 11 to emit light including a red wavelength band (red light Lr). The green phosphor layer 152 absorbs the excitation light EL2 output from the light source unit 11 to emit light including a green wavelength band (green light Lg). The blue phosphor layer 153 absorbs the excitation light EL3 output from the light source unit 11 to emit light including a blue wavelength band (blue light Lb). The IR phosphor layer 154 absorbs the excitation light EL4 output from the light source unit 11 to emit light including an Ir wavelength band (Ir light Lir).

Each of the phosphor layers 151, 152, 153, and 154 includes, for example, what is called a ceramic phosphor formed into a plate shape or a paste binder-type phosphor. The phosphor layers 151, 152, 153, and 154 are respectively formed to include phosphor particles that are respectively excited by the excitation light beams EL1, EL2, EL3, and EL4 output from the respective light source blocks 110R, 110G, 110B, and 110Ir of the light source unit 11 to emit fluorescence light beams having wavelength bands corresponding to red, green, blue, and IR. Examples of the phosphor particles used in the red phosphor layer 151 include CaAlSiN₃:Eu. Examples of the phosphor particles used in the green phosphor layer 152 include Lu₃Al₅O₁₂:Ce. Examples of the phosphor particles used in the blue phosphor layer 153 include (Sr,Ba)₁₀(PO₄)₆Cl₂:Eu. Examples of the phosphor particles used in the IR phosphor layer 154 include LiInSi₂O₆:Cr³⁺. Each of the phosphor layers 151, 152, 153, and 154 may further include semiconductor nanoparticles such as quantum dots, organic dyes, and the like.

The phosphor layers 151, 152, 153, and 154 are respectively provided one by one on the four side surfaces 140S1, 140S2, 140S3, and 140S4 that are opposed to each other vertically and horizontally of the light pipe 14 configuring the light guide path 140. For example, as described above, in a case where one light condenser lens 13 is disposed between the light source unit 11 and the light pipe 14, the light source blocks 110R, 110G, 110B, and 110Ir and the phosphor layers 151, 152, 153, and 154 are disposed in dispositions that are opposite to each other vertically and horizontally. That is, in the present embodiment, the red phosphor layer 151 is provided on the lower side surface 14S1. The green phosphor layer 152 is provided on the left side surface 14S2. The blue phosphor layer 153 is provided on the upper side surface 14S3. The IR phosphor layer 154 is provided on the right side surface 14S4.

It is to be noted that, in a case where, for example, light having a blue wavelength band is used as the excitation light beams EL1, EL2, EL3, and EL4, the blue phosphor layer 153 may be omitted. In this case, it is possible to use the excitation light EL3 applied to a side surface 143S configuring the light guide path 150 as the blue light Lb.

In the light source apparatus 1 of the present embodiment, the excitation light beams EL1, EL2, EL3, and EL4 output from the respective light source blocks 110R, 110G, 110B, and 110Ir of the light source unit 11 and condensed by the light condenser lens 13 respectively irradiate the red phosphor layer 151, the green phosphor layer 152, the blue phosphor layer 153, and the IR phosphor layer 154 provided on the respective side surfaces 140S1, 140S2, 140S3, and 140S4 of the light guide path 140 on the inner side of the light pipe 14.

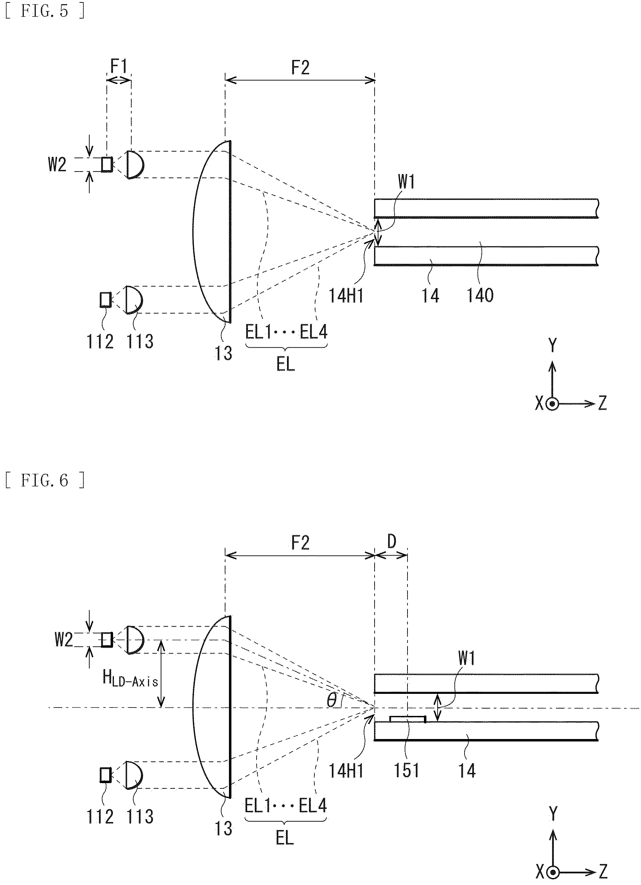

FIG. 5 is an explanatory diagram illustrating the relationship between the size of the opening 14H1 of the light pipe 14 and the spot size of the excitation light EL entering the light pipe 14. In order for the excitation light beams EL1, EL2, EL3, and EL4 output from the respective light source blocks 110R, 110G, 110B, and 110Ir of the light source unit 11 to efficiently irradiate the corresponding phosphor layers 151, 152, 153, and 154, as shown in Expression (1) below, it is preferred that a spot size (H_{LD}) of the excitation light EL entering the light pipe 14 be equal to or smaller than an opening size (W1) of the opening 14H1 of the light pipe 14. It is possible to obtain the spot size (H_{LD}) of the excitation light EL entering the light pipe 14 from, as shown in Expression (2) below, a size (W2) of the light emitting point of the solid-state light emitting device 112, a focal length (F1) of the lens 113, and a focal length (F2) of the light condenser lens 13. $\begin{matrix}W 1 \geq {H}_{LD} \left(1\right) \\ = W 2 \times F 2 / F 1 \left(2\right)\end{matrix}$

As the spot size of the excitation light EL entering the light pipe 14 is smaller , it is possible to cause the excitation light beams EL1, EL2, EL3, and EL4 to selectively irradiate the corresponding phosphor layers 151, 152, 153, and 154. That is, it is desired that the excitation light beams EL1, EL2, EL3, and EL4 be condensed to the opening 14H1 of the light pipe 14.

FIG. 6 is an explanatory diagram illustrating, for example, the red phosphor layer 151 as an example of the position of each of the phosphor layers 151, 152, 153, and 154 disposed in the light guide path 140 of the light pipe 14. In a case where the excitation light beams EL1, EL2, EL3, and EL4 output from the respective light source blocks 110R, 110G, 110B, and 110Ir of the light source unit 11 are condensed to the opening 14H1 of the light pipe 14, it is possible to obtain a position D of the red phosphor layer 151 in the light guide path 140 (a distance D from the opening 14H1 to a center portion of the red phosphor layer 151) from, as shown in Expression (3) below, the opening size (W1) of the opening 14H1 of the light pipe 14 and an incidence angle θ of the excitation light EL1 to the opening 14H1 of the light pipe 14. It is possible to obtain the incidence angle θ of the excitation light EL1 from, as shown in Expression (4) below, an optical axis position (H_{LD-Axis}) of the solid-state light emitting device 112 and the focal length (F2) of the light condenser lens 13. $\begin{matrix}D = W 1 / 2 \div tanθ \left(3\right) \\ θ = arctan \left({H}_{LD - Axis}\div F2\right) \left(4\right)\end{matrix}$

In the light source apparatus 1 of the present embodiment, light beams (red light Lr, green light Lg, blue light Lb, and Ir light Lir) emitted from the respective phosphor layers 151, 152, 153, and 154 by irradiating the phosphor layers 151, 152, 153, and 154 with the respective excitation light beams EL1, EL2, EL3, and EL4 are combined in the light guide path 140 to be extracted as illumination light from the opening 14H2.

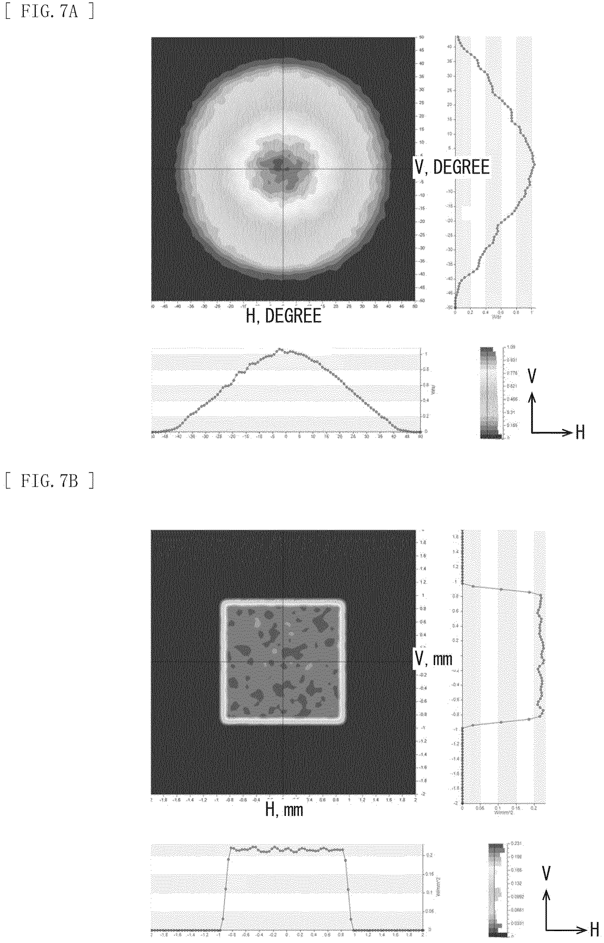

FIG. 7A is an angle distribution diagram of the excitation light beams EL1, EL2, EL3, and EL4 entering the opening 14H1 of the light pipe 14. FIG. 7B is an illuminance distribution diagram of the illumination light that is output from the opening 14H2 of the light pipe 14 to enter the light guide 30. The excitation light beams EL1, EL2, EL3, and EL4 that have entered the light pipe 14 respectively irradiate the phosphor layers 151, 152, 153, and 154. The phosphor layers 151, 152, 153, and 154 respectively emit the red light Lr, the green light Lg, the blue light Lb, and the Ir light Lir. The red light Lr, the green light Lg, the blue light Lb, and the Ir light Lir respectively emitted from the phosphor layers 151, 152, 153, and 154 are repeatedly reflected toward the opening 14H2 on the four side surfaces 140S1, 140S2, 140S3, and 140S4 configuring the light guide path 140. This repeated reflection allows illumination light having a top-hat illuminance distribution as illustrated in FIG. 7B to be extracted from the opening 14H2.

The diffusion plate 21 causes the illumination light output from the light pipe 14 to enter the light guide 30 with the radiation angle of the illumination light being increased.

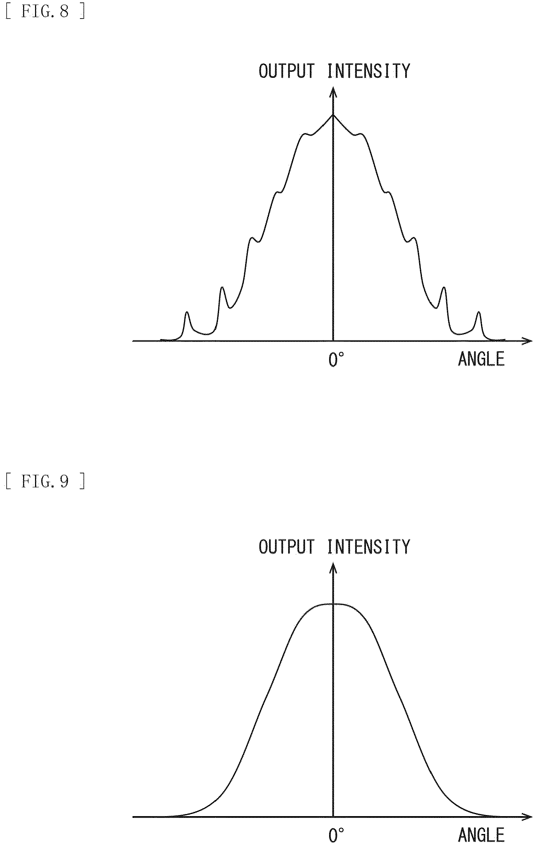

FIG. 8 is an angle distribution diagram of the illumination light output from the opening 14H2 of the light pipe 14. FIG. 9 is an angle distribution diagram of the illumination light output from the opening 14H2 of the light pipe 14 via the diffusion plate 21. In the illumination light output from the opening 14H2, positive and negative signs of an output angle are reversed depending on whether or not the illumination light is reflected at the time of output. The illuminance distribution of the illumination light output from the opening 14H2 thus tends to be discrete as illustrated in FIG. 8. When the diffusion plate 21 is disposed at the opening 14H2 of the light pipe 14, illumination light having a smooth angle distribution as illustrated in FIG. 9 enters the light guide 30.

The light guide 30 allows incident light from the light source apparatus 1 to effectively propagate to the other side, and it is possible to use, for example, an optical fiber.

### [Actions and Effects]

In the light source apparatus 1 of the present embodiment, by using the light source unit 11 that divides excitation light into a plurality of light beams and outputs the plurality of light beams, and the light pipe 14 including, on an inner side thereof, the hollow light guide path 140 and including the red phosphor layer 151, the green phosphor layer 152, the blue phosphor layer 153, and the IR phosphor layer 154 respectively provided on the side surfaces 140S1, 140S2, 140S3, and 140S4 of the light guide path 140, the plurality of light beams (the excitation light beams EL1, EL2, EL3, and EL4) output from the light source unit 11 are caused to respectively irradiate, via the light condenser lens 13, the red phosphor layer 151, the green phosphor layer 152, the blue phosphor layer 153, and the IR phosphor layer 154 provided on the side surfaces of the light guide path 140, and light beams are combined in the light guide path 140. Hereinafter, this action is described.

In recent years, since it is possible to visualize cancer cells by an indocyanine green (ICG) medical agent and IR light irradiation, from the viewpoint of feature increase, IR light is also necessary together with visible light as a light source for medical equipment.

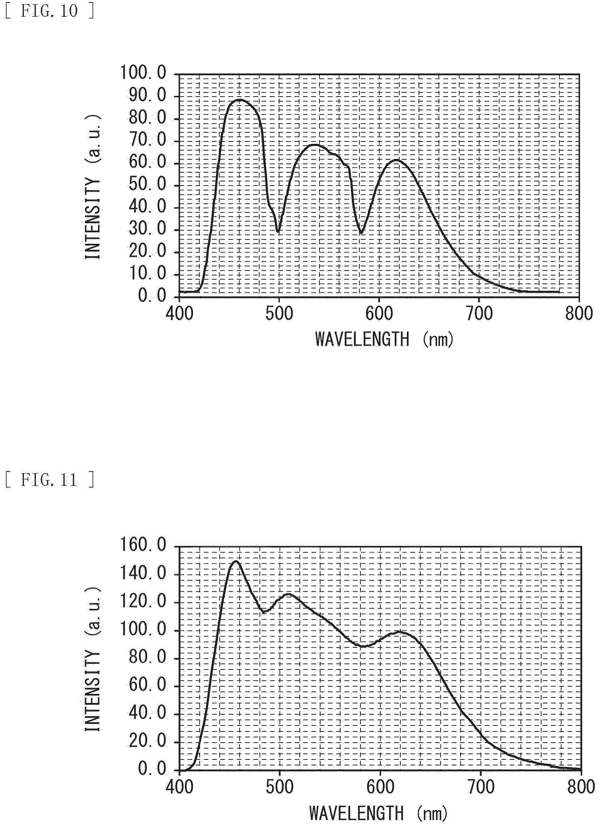

Incidentally, there is a demand for the light source for medical equipment to have an excellent color rendering property. However, illumination light obtained from a general light source apparatus that extracts color light beams respectively corresponding to red (R), greed (G), blue (B), and near infrared (IR) from respective independent wavelength conversion devices to combine the color light beams via a plurality of optical members has had a disadvantage of a low color rendering property. One reason for this is considered to be that, with a wavelength selection device such as a dichroic mirror being interposed in the process of combining the color light beams extracted from the respective wavelength conversion devices, for example, as illustrated in FIG. 10, the wavelength distribution has valleys in the wavelength range of the visible light.

In contrast, in the present embodiment, the illumination light is obtained by using the light source unit 11 that divides the excitation light into the plurality of light beams and outputs the plurality of light beams, the light pipe 14 including, on an inner side thereof, the hollow light guide path 140 and including the red phosphor layer 151, the green phosphor layer 152, the blue phosphor layer 153, and the IR phosphor layer 154 provided on the respective side surfaces 140S1, 140S2, 140S3, and 140S4 of the light guide path 140, and the light condenser lens 13 that is disposed between the light source unit 11 and the light pipe 14 and is configured to condense the excitation light EL output from the light source unit 11 toward the light guide path 140 of the light pipe 14.

In the light source apparatus 1, the light beams (the excitation light beams EL1, EL2, EL3, and EL4) divided and output from the light source unit 11 are condensed by the light condenser lens 13 toward the opening 14H1 of the light pipe 14. The excitation light beams EL1, EL2, EL3, and EL4 condensed to the opening 14H1 of the light pipe 14 respectively irradiate the corresponding phosphor layers (the red phosphor layer 151, the green phosphor layer 152, the blue phosphor layer 153, and the IR phosphor layer 154) respectively provided on the four side surfaces 140S1, 140S2, 140S3, and 140S4 configuring the light guide path 140 on the inner side of the light pipe 14. The light beams (the red light Lr, the green light Lg, the blue light Lb, and the Ir light Lir) emitted from the respective phosphor layers 151, 152, 153, and 154 are combined in the light guide path 140 without passing through a wavelength selection element or the like, and are extracted from the opening 14H2 as the illumination light. This makes it possible to obtain, for example, as illustrated in FIG. 11, illumination light having a wavelength distribution without a valley in the wavelength range of the visible light.

As described above, it is possible for the light source apparatus 1 of the present embodiment to improve the color rendering property.

Further, the light source apparatus 1 of the present embodiment uses the light source unit 11 that divides the excitation light into a plurality of light beams (the excitation light beams EL1, EL2, EL3, and EL4) and outputs the plurality of light beams. Specifically, for example, the plurality of solid-state light emitting devices 112 disposed in an array shape is divided into four light source blocks (the red light source block 110R, the green light source block 110G, the blue light source block 110B, and the IR light source block 110Ir), and the light beams output from the respective light source blocks 110R, 110G, 110B, and 110Ir are used as the excitation light beams EL1, EL2, EL3, and EL4. Moreover, the light source blocks 110R, 110G, 110B, and 110Ir are allowed to be independently controlled by currents. This makes it possible to reflect the intensities of the respective excitation light beams EL1, EL2, EL3, and EL4 to the respective phosphor layers 151, 152, 153, and 154. That is, it is possible to freely adjust the wavelength distribution of light (illumination light) to be extracted from the light pipe 14. Further, it is possible to adjust light deteriorated due to elapse of time.

Moreover, in the light source apparatus 1 of the present embodiment, the red phosphor layer 151, the green phosphor layer 152, the blue phosphor layer 153, and the IR phosphor layer 154 are respectively provided on the side surfaces 140S1, 140S2, 140S3, and 140S4 on the inner side of the light pipe 14 configuring the light guide path 140, and the light beams (the red light Lr, the green light Lg, the blue light Lb, and the Ir light Lir) emitted from the respective phosphor layers 151, 152, 153, and 154 are combined in the light guide path 140. This makes it possible to downsize the apparatus as compared with the general light source apparatus as described above in which color light beams corresponding to red (R), green (G), blue (B), and near infrared (IR) are extracted from the respective independent wavelength conversion devices and the color light beams are combined via a plurality of optical members. Further, it is also possible to reduce the number of components as compared with the above-described general light source apparatus, and hence reduction in cost is also possible.

Still further, in the light source apparatus 1 of the present embodiment, the light pipe 14 is disposed in the stage preceding the light guide 30. In the light guide path 140 of the light pipe 14, the light beams (the red light Lr, the green light Lg, the blue light Lb, and the Ir light Lir) emitted from the respective phosphor layers 151, 152, 153, and 154 are combined while being repeatedly reflected on the side surfaces 140S1, 140S2, 140S3, and 140S4 toward the opening 14H2 to enter the light guide 30 via, for example, the diffusion plate 21. This allows the illuminance distribution of light applied from the light guide 30 to become more uniform. Thus, it is possible to provide a low-etendue light source apparatus 1 having a top-hat illuminance distribution.

Next, Modification Examples 1 to 4 of the present disclosure are described. In the following, components similar to the above-described embodiment are denoted by the same reference symbols, and description thereof is omitted as appropriate.

### <2. Modification Example>

### (2-1. Modification Example 1)

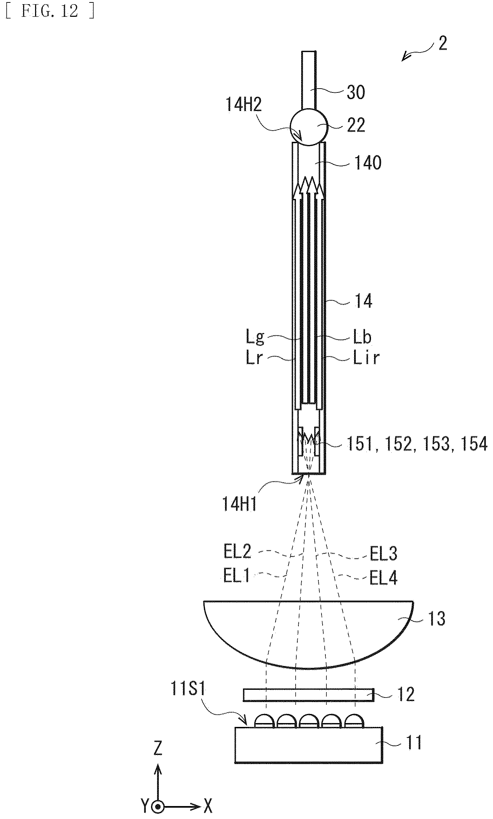

FIG. 12 is a diagram schematically illustrating a configuration example of a light source apparatus (a light source apparatus 2) according to Modification Example 1 of the present disclosure. Similarly to the above-described embodiment, the light source apparatus 2 is used as a light source of medical equipment such as a gastroscopy camera or an endoscope camera.

In the above-described embodiment, description has been given of an example in which, for example, the diffusion plate 21 is disposed at the opening 14H2 of the light pipe 14, but the present disclosure is not limited thereto. In the light source apparatus 2 of this modification example, a ball lens 22 is disposed at the opening 14H2 of the light pipe 14.

The ball lens 22 is a small-diameter spherical single lens having a short focal length and a large numerical aperture (NA). It is possible to mechanically fit the ball lens 22 to the opening 14H2 of the light pipe 14. This makes it possible to automatically adjust the optical axes of the light pipe 14 and the ball lens 22, and hence three-axis adjustment is unrequired.

As described above, in the light source apparatus 2 of this modification example, the ball lens 22 is disposed at the opening 14H2 of the light pipe 14. This makes it possible for the light source apparatus 2 of this modification example to achieve, in addition to the effects of the above-described embodiment, easy adjustment of the angle distribution of the illumination light output from the opening 14H2 of the light pipe 14. In addition, it is possible to contribute to low etendue.

### (2-2. Modification Example 2)

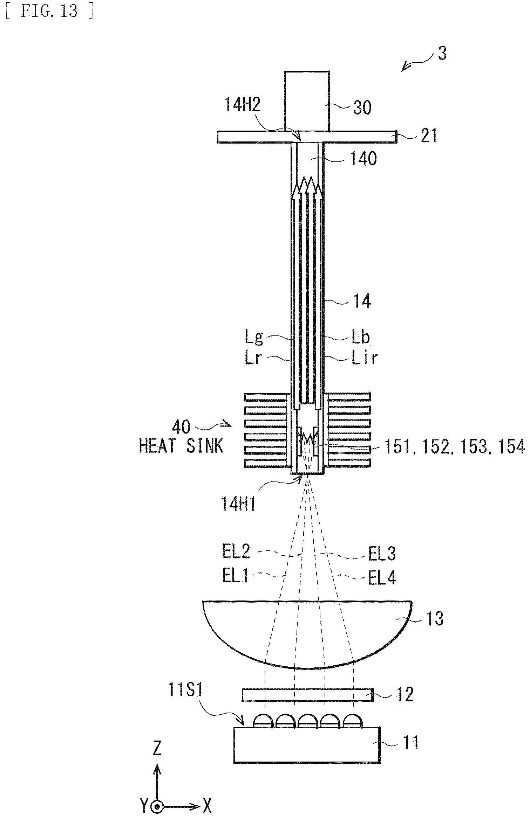

FIG. 13 is a diagram schematically illustrating a configuration example of a light source apparatus (a light source apparatus 3) according to Modification Example 2 of the present disclosure. Similarly to the above-described embodiment, the light source apparatus 3 is used as a light source of medical equipment such as a gastroscopy camera or an endoscope camera.

In the light source apparatus 3 of this modification example, a heat sink 40 is provided on an outer side surface of the light pipe 14.

As described above, in the light source apparatus 3 of this modification example, the heat sink 40 is provided on the outer side surface of the light pipe 14. This makes it possible for the light source apparatus 3 of this modification example to achieve, in addition to the effects of the above-described embodiment, improvement in heat resistance of the light pipe 14. Further, it is possible to reduce deterioration of the phosphor layers 151, 152, 153, and 154 due to heat generation.

### (2-3. Modification Example 3)

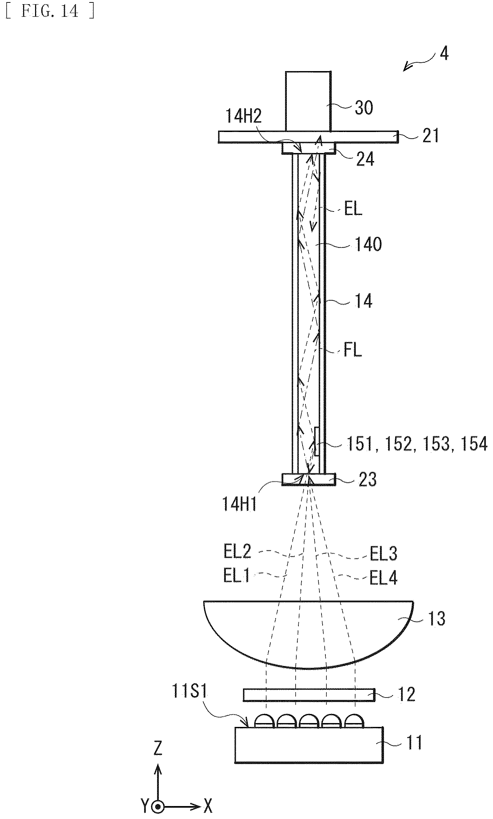

FIG. 14 is a diagram schematically illustrating a configuration example of a light source apparatus (a light source apparatus 4) according to Modification Example 3 of the present disclosure. Similarly to the above-described embodiment, the light source apparatus 4 is used as a light source of medical equipment such as a gastroscopy camera or an endoscope camera.

In the light source apparatus 4 of this modification example, dichroic mirrors 23 and 24 are respectively disposed at the openings 14H1 and 14H2 of the light pipe 14.

The dichroic mirrors 23 and 24 each have a property of selectively reflecting color light having a predetermined wavelength band and transmitting light having other wavelength bands. Specifically, the dichroic mirror 23 disposed at the opening 14H1 of the light pipe 14 selectively transmits excitation light beams EL (EL1, EL2, EL3, and EL4) divided and output from the light source unit 11, and selectively reflects fluorescence light beams FL emitted from the respective phosphor layers 151, 152, 153, and 154. The dichroic mirror 24 disposed at the opening 14H2 of the light pipe 14 selectively reflects the excitation light beams EL (EL1, EL2, EL3, and EL4) divided and output from the light source unit 11, and selectively transmits the fluorescence light beams FL emitted from the respective phosphor layers 151, 152, 153, and 154.

As described above, in the light source apparatus 4 of this modification example, the dichroic mirrors 23 and 24 are respectively disposed at the openings 14H1 and 14H2 of the light pipe 14. This causes the excitation light EL that has reached the opening 14H2 without being subjected to wavelength conversion in each of the phosphor layers 151, 152, 153, and 154 to return into the light guide path 140 by the dichroic mirror 24, and causes the fluorescence light FL emitted to the opening 14H1 side from each of the phosphor layers 151, 152, 153, and 154 to return into the light guide path 140 by the dichroic mirror 23. It is thus possible for the light source apparatus 4 to achieve improvement in optical efficiency in addition to the effects of the above-described embodiment.

### (2-4. Modification Example 4)

In the above-described embodiment, description has been given of an example in which the phosphor layers 151, 152, 153, and 154 are disposed at the same positions from the opening 14H1 of the respective four side surfaces 140S1, 140S2, 140S3, and 140S4 configuring the light guide path 140, but the present disclosure is not limited thereto.

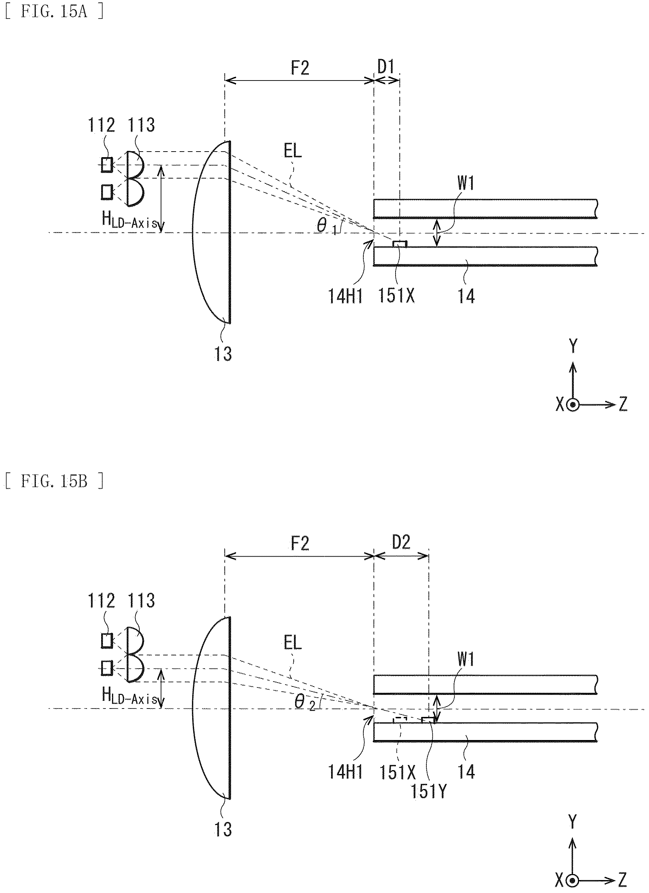

The positions of the respective phosphor layers 151, 152, 153, and 154 may be changed as illustrated in FIG. 15A and FIG. 15B on the basis of Expression (3) and Expression (4) described above, in the longitudinal direction of the light guide path 140 (for example, from a phosphor layer 151X of FIG. 15A to a phosphor layer 151Y of FIG. 15B) in accordance with the incidence angle θ of the excitation light EL and the optical axis position (H_{LD-Axis}) of the solid-state light emitting device 112.

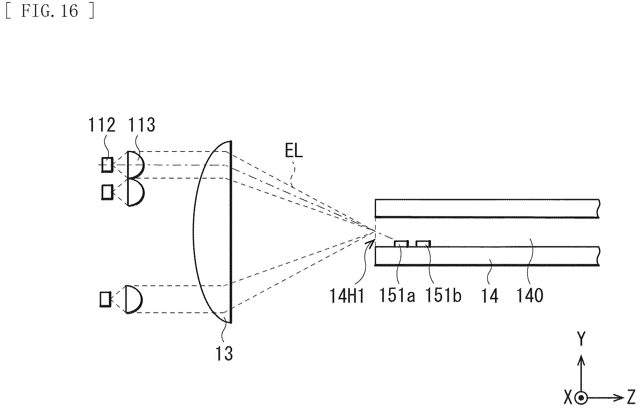

Further, the phosphor layers 151, 152, 153, and 154 may be changed in the concentration of the phosphor particles included in the layers, in the longitudinal direction of the light guide path 140 (Z-axis direction). As another example, as illustrated in FIG. 16, a plurality of phosphor layers 151a and 151b having a difference in the concentration of the phosphor particles may be disposed in the longitudinal direction (Z-axis direction). Moreover, in a case where the plurality of phosphor layers is disposed in the longitudinal direction of the light guide path 140, phosphor particles having different light emitting wavelengths may be used for the phosphor layers 151a and 151b.

As described above, the positions of the respective phosphor layers 151, 152, 153, and 154 in the light guide path 140 are changed, a concentration gradient of the phosphor particles is formed in the layers of the respective phosphor layers 151, 152, 153, and 154 or in the plurality of phosphor layers 151a and 151b disposed in the longitudinal direction, or the plurality of phosphor layers (for example, the phosphor layers 151a and 151b) disposed in the longitudinal direction is formed by using phosphor particles having different light emitting wavelengths. This makes it possible to finely adjust the chromaticity of the illumination light.

The present technique has been described above by means of the embodiment and Modification Examples 1 to 4, but the present technique is not limited to the above-described embodiment and others, and various modifications may be made thereto. For example, the disposition, number, and the like of components of an optical system exemplified in the above-described embodiment and others are merely examples. Not all of the components are required to be included, and other components may further be included.

Further, the light source apparatus 1 to 4 of the present technique may be used in electronic equipment other than medical equipment. For example, the light source apparatus 1 to 4 may be used for the purpose of illumination, and is applicable to, for example, a headlamp of a vehicle or a light source for lighting-up.

It is to be noted that the effects are not always limited those described herein and may be any effect described in the present disclosure.

It is to be noted that the present technique may take the following configuration. According to the present technique having the following configuration, by using the light source unit configured to output the plurality of divided light beams and the light guide member including, on the inner side thereof, the hollow light guide path having the first opening and the second opening on a side opposite to the first opening and having the plurality of wavelength converters that emits light beams having wavelength bands different from each other provided in at least a portion of the side surface of the light guide path, each of the plurality of divided light beams output from the light source unit as the excitation light is applied via the light condenser device to a corresponding one of the plurality of wavelength converters provided on the inner side of the light guide member. This makes it possible to extract light having a desired wavelength distribution, and to achieve a high color rendering property.
(1)
   A light source apparatus including:
   a light source unit that divides excitation light into a plurality of light beams and outputs the plurality of light beams;
   a light guide member including, on an inner side thereof, a hollow light guide path having a first opening and a second opening on a side opposite to the first opening, the light guide member having a plurality of wavelength converters provided on at least a portion of a side surface of the light guide path, the plurality of wavelength converters being configured to emit light beams having wavelength bands different from each other; and
   a light condenser device that guides each of the plurality of light beams output from the light source unit to a corresponding one of the plurality of wavelength converters.
(2) The light source apparatus according to (1), in which the light source unit divides the excitation light into as many light beams as the plurality of wavelength converters and outputs the plurality of light beams.
(3) The light source apparatus according to (1) or (2), in which
   the plurality of wavelength converters includes a first wavelength converter that performs wavelength conversion of the excitation light into light of a red band, a second wavelength converter that performs wavelength conversion of the excitation light into light of a green band, a third wavelength converter that performs wavelength conversion of the excitation light into light of a blue band, and a fourth wavelength converter that performs wavelength conversion of the excitation light into light of a near infrared region, and
   the light source unit includes a first light source block that irradiates the first wavelength converter, a second light source block that irradiates the second wavelength converter, a third light source block that irradiates the third wavelength converter, and a fourth light source block that irradiates the fourth wavelength converter.
(4) The light source apparatus according to (3), in which the first light source block, the second light source block, the third light source block, and the fourth light source block are independently controlled by currents.
(5) The light source apparatus according to (3) or (4), in which
   the light guide member has, on the inner side, four side surfaces that are opposed to each other vertically and horizontally, and
   any one of the first wavelength converter, the second wavelength converter, the third wavelength converter, and the fourth wavelength converter is provided on a corresponding one of the four side surfaces.
(6) The light source apparatus according to any one of (1) to (5), in which the light guide member includes a light pipe.
(7) The light source apparatus according to any one of (1) to (6), further including a ball lens disposed at the second opening of the light guide member.
(8) The light source apparatus according to any one of (1) to (7), further including a heat sink disposed on an outer side surface of the light guide member.
(9) The light source apparatus according to any one of (1) to (8), further including a first dichroic mirror disposed at the first opening of the light guide member, the first dichroic mirror transmitting the excitation light and reflecting light subjected to wavelength conversion in the plurality of wavelength converters.
(10) The light source apparatus according to any one of (1) to (9), further including a second dichroic mirror disposed at the second opening of the light guide member, the second dichroic mirror reflecting the excitation light and transmitting light subjected to wavelength conversion in the plurality of wavelength converters.
(11) The light source apparatus according to any one of (1) to (10), in which
   each of the plurality of wavelength converters includes a phosphor, and
   each of the plurality of wavelength converters has a concentration gradient of the phosphor in a longitudinal direction of the light guide member.
(12) The light source apparatus according to any one of (1) to (11), in which each of the plurality of wavelength converters includes, in a longitudinal direction of the light guide member, a plurality of wavelength conversion blocks having conversion wavelengths different from each other within a predetermined wavelength range.
(13) The light source apparatus according to any one of (1) to (12), in which
   the light guide member has, on the inner side, four side surfaces that are opposed to each other vertically and horizontally, and
   any one of the plurality of wavelength converters is provided on a corresponding one of three side surfaces out of the four side surfaces, and none of the plurality of wavelength converters are provided on one side surface out of the four side surfaces.
(14) The light source apparatus according to any one of (1) to (13), further including a diffusion plate on the second opening side of the light guide member.

The present application claims the benefit of Japanese Priority Patent Application JP2023-051786 filed with the Japan Patent Office on March 28, 2023, the entire contents of which are incorporated herein by reference.

It should be understood by those skilled in the art that various modifications, combinations, sub-combinations and alterations may occur depending on design requirements and other factors insofar as they are within the scope of the appended claims or the equivalents thereof.

## Claims

1. A light source apparatus comprising:
a light source unit that divides excitation light into a plurality of light beams and outputs the plurality of light beams;
a light guide member including, on an inner side thereof, a hollow light guide path having a first opening and a second opening on a side opposite to the first opening, the light guide member having a plurality of wavelength converters provided on at least a portion of a side surface of the light guide path, the plurality of wavelength converters being configured to emit light beams having wavelength bands different from each other; and
a light condenser device that guides each of the plurality of light beams output from the light source unit to a corresponding one of the plurality of wavelength converters.

2. The light source apparatus according to claim 1, wherein the light source unit divides the excitation light into as many light beams as the plurality of wavelength converters and outputs the plurality of light beams.

3. The light source apparatus according to claim 1, wherein
the plurality of wavelength converters includes a first wavelength converter that performs wavelength conversion of the excitation light into light of a red band, a second wavelength converter that performs wavelength conversion of the excitation light into light of a green band, a third wavelength converter that performs wavelength conversion of the excitation light into light of a blue band, and a fourth wavelength converter that performs wavelength conversion of the excitation light into light of a near infrared region, and
the light source unit includes a first light source block that irradiates the first wavelength converter, a second light source block that irradiates the second wavelength converter, a third light source block that irradiates the third wavelength converter, and a fourth light source block that irradiates the fourth wavelength converter.

4. The light source apparatus according to claim 3, wherein the first light source block, the second light source block, the third light source block, and the fourth light source block are independently controlled by currents.

5. The light source apparatus according to claim 3, wherein
the light guide member has, on the inner side, four side surfaces that are opposed to each other vertically and horizontally, and
any one of the first wavelength converter, the second wavelength converter, the third wavelength converter, and the fourth wavelength converter is provided on a corresponding one of the four side surfaces.

6. The light source apparatus according to claim 1, wherein the light guide member comprises a light pipe.

7. The light source apparatus according to claim 1, further comprising a ball lens disposed at the second opening of the light guide member.

8. The light source apparatus according to claim 1, further comprising a heat sink disposed on an outer side surface of the light guide member.

9. The light source apparatus according to claim 1, further comprising a first dichroic mirror disposed at the first opening of the light guide member, the first dichroic mirror transmitting the excitation light and reflecting light subjected to wavelength conversion in the plurality of wavelength converters.

10. The light source apparatus according to claim 1, further comprising a second dichroic mirror disposed at the second opening of the light guide member, the second dichroic mirror reflecting the excitation light and transmitting light subjected to wavelength conversion in the plurality of wavelength converters.

11. The light source apparatus according to claim 1, wherein
each of the plurality of wavelength converters includes a phosphor, and
each of the plurality of wavelength converters has a concentration gradient of the phosphor in a longitudinal direction of the light guide member.

12. The light source apparatus according to claim 1, wherein each of the plurality of wavelength converters includes, in a longitudinal direction of the light guide member, a plurality of wavelength conversion blocks having conversion wavelengths different from each other within a predetermined wavelength range.

13. The light source apparatus according to claim 1, wherein
the light guide member has, on the inner side, four side surfaces that are opposed to each other vertically and horizontally, and
any one of the plurality of wavelength converters is provided on a corresponding one of three side surfaces out of the four side surfaces, and none of the plurality of wavelength converters are provided on one side surface out of the four side surfaces.

14. The light source apparatus according to claim 1, further comprising a diffusion plate on the second opening side of the light guide member.
